Europäisches Patentamt

European Patent Office

Office européen des brevets

(11) Publication number: **0 418 449 A1**

# EUROPEAN PATENT APPLICATION

(21) Application number: **89600016.3**

(22) Date of filing: **20.09.89**

(51) Int. Cl.5: **A61N 2/02**

(30) Priority: **20.09.89 GR 89010595**

(43) Date of publication of application:
**27.03.91 Bulletin 91/13**

(84) Designated Contracting States:
**AT BE CH DE ES FR GB GR IT LI LU NL SE**

(71) Applicant: **Pappas, Panagiotis Th.**
**Marcopulioti 26**
**GR-11744 Athens(GR)**

Applicant: **Cure, Jorge**
**8201 NW 191 Lane**
**Miami Florida 33015(US)**

Applicant: **Eichler, Harry**
**5323 Hayes Street**
**Hollywood Florida 33021(US)**

(72) Inventor: **Pappas, Panagiotis Th.**
**Marcopulioti 26**
**GR-11744 Athens(GR)**
Inventor: **Cure, Jorge**
**8201 NW 191 Lane**
**Miami Florida 33015(US)**
Inventor: **Eichler, Harry**
**5323 Hayes Street**
**Hollywood Florida 33021(US)**

(74) Representative: **Sakellarides, John A.**
**6, Heraklitou Street**
**GR-106 73 Athens(GR)**

(54) **Ion dispersing device by remote action.**

(57) The invention concerns a device that by remote action disperses electric charges or ionic concentrations inside organic or inorganic matter, inside biological matter, or inside any matter containing mobile electric charges. The charge transport takes place by remote action, without direct contact and with the main characteristic, a particular direction or one way motion of the charges from a particular target region. Therefore, the device is useful for charging or discharging regions inside bulk matter, where electrical contact is not easy or is not possible. The device is ideal for the Nordenstrom method in the field of medicine, where electrical circuits are established between particular regions, without the necessary electrode invasion.

FIG. 1

EP 0 418 449 A1

## ION DISPERSING DEVICE BY REMOTE ACTION

Recently, in biology and medicine there has been increased interest in the action of ionic concentrations in and around cells. (References 1 to 11). Some years ago, it was suggested by Szent-Gyorgyi, (references 1,2,3), that if D/A = 1, D signifying positive ions, electron donor molecules, and A signifying negative ions, electron acceptor molecules, in the vicinity of cells, then the cells are in equilibrium. On the other hand, if D/A >1, then the cells have the tendency to proliferate without control, causing cancer. This hypothesis was later confirmed, (references 3 and 7), by the introduction of chemical substances, (chemotherapy), in the region of cancerous cells, such chemical substances being natural donors D or natural acceptors A. It was observed that the presence of substances of category A diminish the growth of cancerous cells, i.e. chemical substances with A signature are carcinostatic. On the contrary, the introduction by chemotherapy of substances with D signature, cause no substantial difference to the already proliferating cancerous cells, which as a rule are located in D environment, in contrast to the healthy, equilibrated cells for which D/A = 1. The same fact was confirmed by Cone (references 4,5,6) by direct measurements of the electrical potential inside and outside of cancerous cells.

Nordenstrom, working along the same lines, was able to develop his own method for treating cancer, by directly introducing electrical probes into the cancerous regions to disperse the positive ions, D. Other than the applied chemotherapy with acceptors A, and the Nordenstrom method, there does not seem to exist any other method for neutralizing the donor substances D in the region of cancerous cells. The dispersion or concentration of donors D by electrostatic fields seems to have undetectable effects. An electrostatic field does not generate a detectable electric current, other than momentary charge transport which is insufficient for an electrolytic dispersion or neutralization of ions. On the other hand, the effect of alternating electrical fields seem to have no substantial results, because of the alternating dispersion and concentration of the donors D to and from a target region. The use of static magnetic fields is known to have no effect on static distributions of charges or ions. Alternating magnetic fields produce effects similar to alternating electrical fields, alternating dispersion and concentration of electrical charges. The resulting alternating electromotive force $E = kdB/dt = k'di/dt$ causes no net effect as far as ion dispersion or ion concentrations in a target region. As far as the dispersion or concentration of charges is concerned, alternating electromagnetic fields or waves of any frequency appear to have the same results as the above results for alternating electric or alternating magnetic fields of the same frequency. Beyond these two methods; - a. chemotherapy, furnishing of acceptors orally or by injection: b. the direct electric conduction of the Nordenstrom method, which in most cases requires bodily invasion, i.e. an operation to facilitate electrode entrance and contact to the target region, provided the operation is possible - there seems to be no other known method for controlling the ions D and A inside biological matter. Both methods seem to have serious disadvantages. On one hand, chemotherapy has the various known side effects and its results are often doubtful. On the other hand, in the case of the Nordenstrom method, there is the serious consequences having an operation, if such an operation is possible, besides, in some cases electrode invasion may not permitted at all.

The present invention gives an effective solution to the above problem, without the described disadvantages. In addition, this method can be applied in general to other situations (other than medicine), which require systematic unidirectional charge transport (substantial direct current), and for which there is no easy access for direct electrical contact.

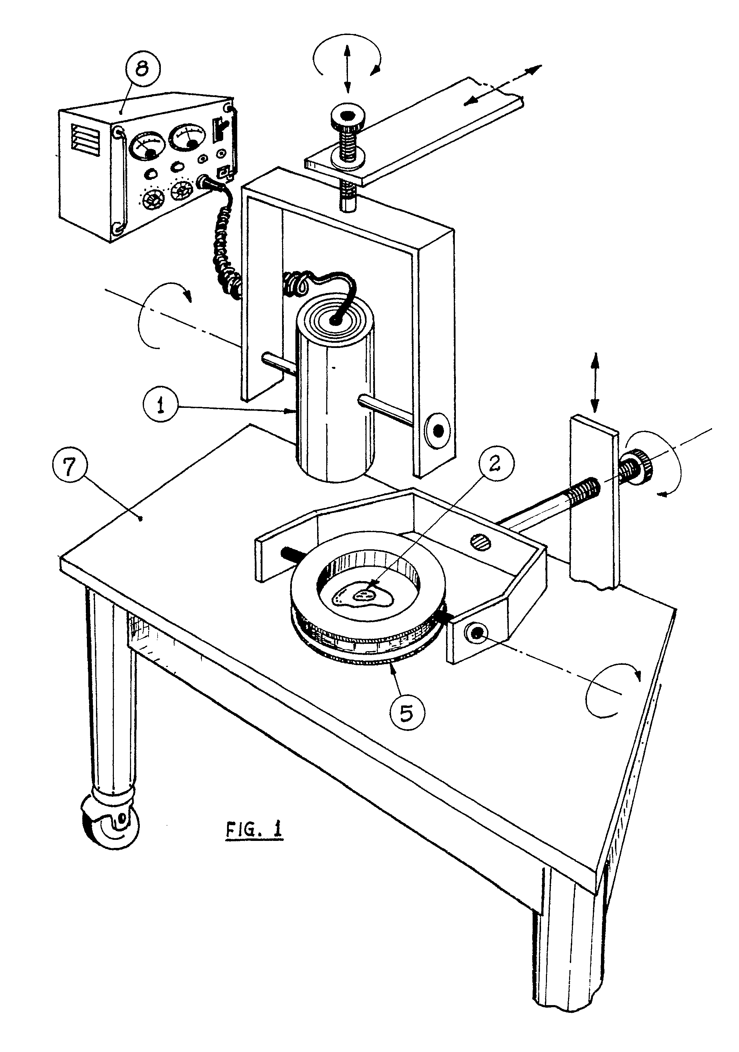

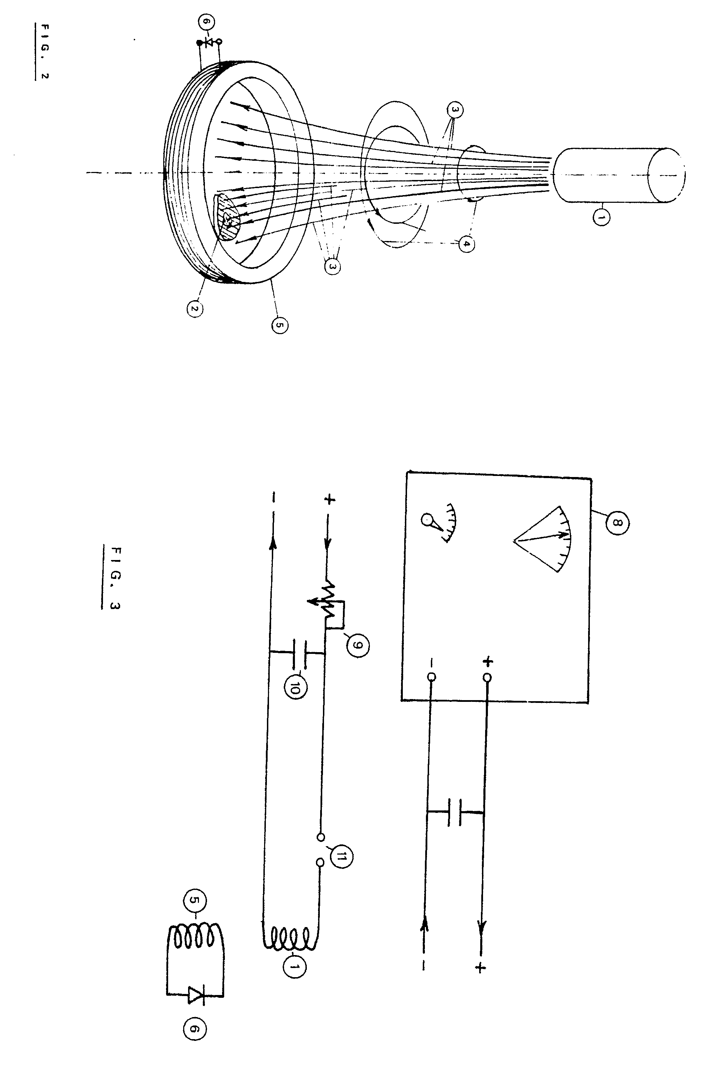

The description of the invention is as follows: The invention consists of a main coil (1), mounted on a moving arm, to which an interrupted current is supplied with a fast rise time, i.e., a square or triangular pulse, or another appropriate pulse from a particular electric source (8), or even the current discharged from a charged capacitor, etc. Below or above the coil (1), a moving table made of insulating material is placed, on top of which the material or the object containing the region (2) in which ions are to be dispersed; see Figure 1. In this way coil (1) is located in such a way that its magnetic lines B (3) cross through region (2), or in other words region (2) is placed close to the perimeter of the concentric magnetic potential A (4) of the coil (1); see figures 1 and 2. A second coil (5) also mounted on a moving arm, surrounds region (2), such that the coil's plane (5) cuts the magnetic lines B (3) in a perpendicular fashion, i.e., the turns of the coil (5) are parallel to the magnetic potential A (4); see Figures 1 and 2. Coil (5) is short-circuited with a fast-recovery, high-tension and high-power diode (6). The polarity of diode (6) depends on the direction of the current which we wish to induce in region (2). The current in region (2) is opposite to the current in the coil (5), which corresponds to the conducting current in the diode (6). The polarity of coil (1) is chosen in such a way as to have its initial current opposed to the desired current in region (2).

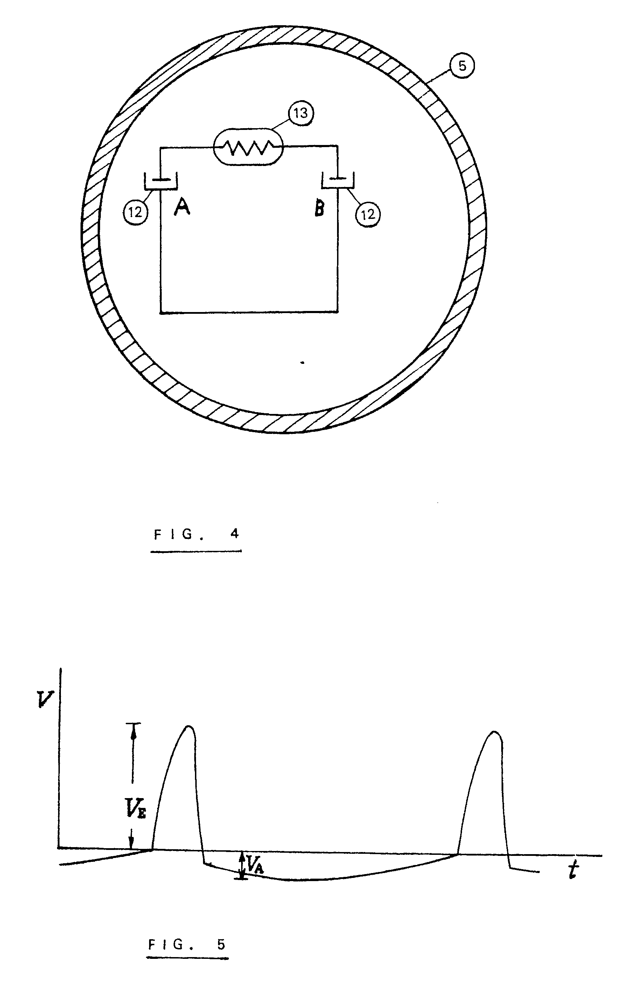

The function of the device is as follows. When a high surge voltage is supplied to the coil, a fast-rising current flows. At the same time an induced current appears on every conducting surface or in every conducting volume, that opposes the increase of the magnetic flux in coil (1), according to Lenz rule of electromagnetism. During this phase, coil (5) remains inactive, because its diode (6) is polarized not to conduct. However, ions are displaced from the target region (2) towards a particular direction. Immediately after this, and as a rule, the current in coil (1) will be set into oscillation at a frequency that depends on its natural resonance. During those oscillations, the magnitude of the current in coil (1) will be decreasing. Coil (5) will start conducting a current to maintain the initial maximum of the magnetic flux, according to the rule of Lenz. In this way, coil (5) considerably delays the fall of the initial flux through region (2), while coil (1) continues to execute dumping oscillations. Finally, the magnetic flux through region (2) will fall to zero after a time which depends mainly on the self-inductance and resistance of the coil (5) : $t \approx L/R$. In this way choosing a small self-inductance $L$ for coil (1), a power supply of a high surge voltage (with low internal resistance), a high self-inductance $L$ and low resistance $R$ for coil (5), we may induce (for a short duration) a high voltage $V_E$ in region (2) and in a particular direction, and also any small induced voltage $V_A$ in the opposite direction (for a longer duration), according to the diagram of Figure 5. In this way, we may transfer an amount of charge in the direction determined by the high-magnitude, short-duration induced potential $V_E$, and the same time greatly limit the return flow of the charges in the opposite direction, provided that we keep the small-magnitude, long-duration, oppositely induced potential $V_A$, below the threshold electrolytic potential which confined the initial charge (or ion) concentration. In general, we expect the device to operate effectively, as a "dc inductor" or "dc transformer", so to speak, with respect to any nonlinearly conducting medium, (or medium with non-constant ohmic resistance), by appropriately choosing the parameters of the coils (1) and (5), as well as the voltage and internal resistance of the power supply. The amount of transported charges increases with the repetition of the function cycle of the device, i.e. by repeating the voltage surges in coil (1). Finally the quantity of charge transported or dispersed is a function of the time over which the device operates.

Description of a particular model of the invention: The electrical diagram of a working model of the device is given in the Figure 3. The power supply (8) consists of an adjustable voltage source of 500 to 16000 volts and an 1 μf capacitor filter of 20 kv. The power supply is connected via a rheostat (9), to the main device which consists of a specially constructed capacitor bank (10), with the following specifications: very fast discharge time, very high power, very low internal resistance and parasitic self-inductance, made of two copper sheets 0.3 mm thickness, 1 m² area and separated by a sheet of glass of 3 mm thickness and of the same area, as well as a spark gap (11) with an adjustable gap distance, connected as shown in the diagram of Figure 3. Coil (1) consists of several turns of 40 cm diameter of insulated copper wire of 4 mm diameter. Coil (5) consists of about 1000 turns of 80 cm diameter of enamel copper wire of 2 mm.

The sample object which was used in one of the trials for dispersing its ions consisted of two small uncharged capacitors A and B (12) of 1 μf, connected via a nonlinear resistor or varistor (13), as shown in Figure 4, and placed inside coil (5) which was at 50 cm distance from coil (1). The power supply was adjusted to charge the capacitor (10), so as to cause an arc discharge in the spark gap (11) approximately every 0.3 sec. After 3 min of operating the device, capacitors (12) A and B were immediately measured with a voltmeter. Capacitor A was found charged in the forward direction by several tenths of a volt and capacitor B in the reversed direction by several hundreds of a volt. The difference in the charges is explained by the expected dielectric leakage of capacitor B, charged in its reversed direction. Removing coil (5) or the diode (6) no systematic charge was found in the capacitors A and B, contrary to what was observed before. The difference was distinct and considerable. Inverting the polarity of diode (6) in the coil (5) the charges in the capacitors A and B were always found to be inverted. (The induced charge polarity was characteristic of the device and not of the sample circuit, excluding the possibility of rectification taking place in the sample circuit itself. In later improved tests, plus or minus potential differences of the order of 14 volts were measured in the 1 μf capacitor A, in less than a few seconds of operation of the device. The polarity of the induced potential difference depended solely on the polarity of the external diode (6)).

The advantages of the invention are the following: The main function of the invention is to induce a potential of the form shown in Figure 5. To produce this form by means of another electronic method, it does not seem to be possible or easy, because a considerably large power of several Kwatts is required for substantial results in practice. In such a case, any coil which would have been powered to produce a predetermined form of magnetic flux, would carry parasitic oscillations or would have produced unwanted components of tension in the induced potential. Mainly, these unwanted side effects are suppressed by the innovation of coil (5) with the short-circuited diode (6). The effectiveness of coil (5) and diode (6) is so big that any form of magnetic flux which coil (1) may produce, the induced potential in region (2) is almost always of the desired form shown in Figure 5. Even coil (1) may be powered by a perfect sinusoidal tension of a definite frequency (i.e. by a power from electrical mains, or by a power of a much higher frequency than the mains), for avoiding nearby unwanted interference, with satisfactory results for the induced tension in the target region (2).

Partial advantages of the present device are the following: The induced tension, Figure 5, appears only near the target region (2) and not all over the effective space surrounding coil (1), causing unwanted DC induction to the device operators or to other objects outside region (2). The power which coil (5) and diode (6) absorb is a small fraction of the power of coil (1) (of several Kwatts). In this way, coil (5) and diode (6) with the high requirements described above, may easily cope with the stress of a much lower power. On the other hand, the field of coil (1) is modified to the minimum required degree, which in the case of a sinusoidal power supply, will cause minimum interference. Finally with respect to particular applications for transporting or dispersing ions, the present device is clearly superior for it requires no invasion or entrance to region (2), for example, using electrodes or chemical substances. The expected applications are extended to a large spectrum of technology and science, where charge or ion remote transport is required to occur in not easily accessible regions, in biology, medicine, chemical industry (ion separation), water purification, electrolysis, electrolytic metal plating, electrolytic casting, remote direct current energy supply without contacts, or in general, where a DC transformer or a DC inductor is required.

REFERENCES.

1. Szent-Gyorgyi, A., Introduction to a Submolecular Biology ( Academic Press, N.Y., 1960 )

2. Szent-Gyorgyi, A. Bioelectronics (Academic Press, N.Y., 1968)

3. Szent-Gyorgyi, A., Electronic Biology ( Marcel Dekker, Inc., N.Y., 1976)

4. Cone, C.D., J. Theoret. Biol., 30 , 151-181 (1971)

5. Cone, C.D. Ann. New York Acad. Sci., 238 ,

420-435 (1974)

6. Cone, C.D. Transmembrane Potentials and Characteristics of Immune and Tumor Cells CRC Press, Inc., Boca Raton, Fl., 1985) Ch. 9, p. 138-141

7. Albert, B., Bray, D., Lewis, J., Raff, M., Roberts, K., and Watson, J.D., Molecular Biology of the Cell (Garland Publishing, Inc., NY, 1983)

8. Nordenstrom, B., Biologically Closed Electric Circuits 25 (Nordic Medical Publications, Stockholm, 1983)

9. Barothy, J.M., Biological Effects of Magnetic Fields (Plenum Press, IY, 1964) p. 100-108

10. Cure' J.C. Phys. Lett., 116B , no.2.3, 158-160 (1982)

11. Cure', J.C. Northeastern University, Boston, Nov. 1987 (Private Communication)

## Claims

1. A device for removing ions and/or electrical charges inside materials by remote action, by using: a diode (6) short circuited coil (5) for eliminating and/or smoothing of the negative (decreasing) phase of a magnetic flux; as well as a coil (1) as the source of the magnetic flux, employing any type of electrical power source.

2. A device for removing ions and/or electrical charges inside materials by remote action, in the case in which the above coils (1) and (5) are placed together and/or the coils form one coil and/or one of the coils is part of the other.

3. A device for removing ions and/or electrical charges inside materials by remote action, by using a version of the above device, i.e. a device which consists of the above elements once or more than once, and/or an equivalent version of rectifying diode (6) made of another element or elements which are themselves equivalent rectifiers.

4. A version of the above device which consists of the above elements and/or their parts, in any combination, other secondary elements, and/or a replacement of the coil (1) by any other form of magnetic flux, while the version of the device incorporates the principle of eliminating and/or smoothing of the negative (decreasing) phase of the magnetic flux of the source, by any combination of coils and/or rectifiers in place of the coil (5) and/or rectifier (6).

FIG. 1

FIG. 2

FIG. 3

FIG. 4

FIG. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-182160 (DABER)<br>* the whole document * | 1-4 | A61N2/02 |
| X | EP-A-84019 (LKH AG)<br>* page 4, last paragraph – page 5, paragraph 1; figure * | 1, 2, 4 | |
| X | JOURNAL OF PHYSICS E. SCIENTIFIC INSTRUMENTS. vol. 18, no. 1, January 1985, ISHING, BRISTOL GB pages 75 – 78; D.McROBBIE: "Design and instrumentation of a magnetic nerve stimulator"<br>* page 74; figure 1 *<br>* page 76, right-hand column * | 1, 2, 4 | |
| X | GB-A-2156679 (WALPOLE)<br>* the whole document * | 1, 4 | |

TECHNICAL FIELDS SEARCHED (Int. Cl.5 )

A61N

The present search report has been drawn up for all claims

| Place of search | Date of completion of the search | Examiner |
|---|---|---|
| THE HAGUE | 25 MAY 1990 | LEMERCIER D.L.L. |

EPO FORM 1503 03.82 (P0401)